# NEUE EUROPÄISCHE PATENTSCHRIFT

(11) **EP 2 922 598 B2**
(45) Veröffentlichungstag und Bekanntmachung des Hinweises auf die Entscheidung über den Einspruch: **16.11.2022**
(45) Hinweis auf die Patenterteilung: 09.01.2019
(21) Anmeldenummer: 13789325.1
(22) Anmeldetag: 12.11.2013
(51) Int. Cl.: A61Q 19/00, A61K 8/92, A61K 8/37, A61K 8/81, A61K 8/04

(54) **SENSORISCH ATTRAKTIVE HYDRODISPERSION MIT WACHSEN**
SENSORILY ATTRACTIVE HYDRODISPERSION HAVING WAXES
HYDRODISPERSION SENSORIELLEMENT ATTRACTIVE CONTENANT DES CIRES

(30) Priorität: 20.11.2012 DE 102012221227
(43) Veröffentlichungstag der Anmeldung: 30.09.2015
(73) Patentinhaber: Beiersdorf AG, 20253 Hamburg (DE)
(72) Erfinder: RATSCHOW, Cecile, 22761 Hamburg (DE); MEYER, Christiane, 20357 Hamburg (DE); UHLEN, Janina, 20249 Hamburg (DE); LERG, Heike, 22303 Hamburg (DE); NISSEN, Bente, 20144 Hamburg (DE)
(74) Vertreter: Beiersdorf AG
(86) Internationale Anmeldenummer: PCT/EP2013/073586
(87) Internationale Veröffentlichungsnummer: WO 2014/079734

(56) Entgegenhaltungen:
- EP-A1- 1 576 949
- EP-A1- 1 889 596
- EP-A2- 1 586 307
- WO-A1-2005/020940
- WO-A1-2005/097057
- WO-A2-2005/097055
- US-A1- 2011 250 148

## Beschreibung

Die Erfindung umfasst Hydrodispersion mit Verdickungsmitteln, Wachse und Ölen. Die Wachse und die Gesamtzubereitung weisen spezifische Schmelzbereiche auf, die für ein angenehmes, sensorisch leichtes Profil und gute Pflegeeffekte sorgen.

Häufige Erscheinungsformen kosmetischer oder dermatologischer Zubereitungen sind feindisperse Mehrphasensysteme, in welchen eine oder mehrere Fett- bzw. Ölphasen neben einer bzw. mehreren Wasserphasen vorliegen. Von diesen Systemen sind wiederum die eigentlichen Emulsionen die am weitesten verbreiteten.

Aber auch emulgatorarme bzw. -freie Zubereitungen auf Basis sogenannter Hydrodispersionen sind bekannt. Hydrodispersionen stellen Dispersionen einer flüssigen, halbfesten oder festen inneren (diskontinuierlichen) Lipidphase in einer äußeren wäßrigen (kontinuierlichen) Phase dar. Hydrodispersionen sind - wie auch Emulsionen, die sich durch eine ähnliche Phasenanordnung auszeichnen - metastabile Systeme und daher geneigt, in einen Zustand zweier in sich zusammenhängender diskreter Phasen überzugehen. In einer klassischen O/W-Emulsion verhindert die Wahl eines geeigneten Emulgators die Phasentrennung. Im Gegensatz zur klassischen Emulsionen enthalten Hydrodispersionen aber nur sehr geringe Mengen an Emulgatoren (bis zu 2 Gew.-%) bzw. können sogar gänzlich frei von Emulgatoren sein.

Bei Hydrodispersionen, welche aus einer flüssigen Lipidphase in einer äußeren wässrigen Phase bestehen, wird die Stabilität des Mehrphasensystems üblicherweise dadurch gewährleistet, dass in der wässrigen Phase mit Hilfe eines Gelbildners bzw. Verdickungsmittels ein Gelgerüst aufgebaut wird, in welchem die Lipidtröpfchen stabil suspendiert sind.

Als Verdickungsmittel sind Homopolymere oder Copolymere von Acrylsäure und/oder Acrylamid und deren Derivaten bekannt. Formulierungen auf Polyacrylatbasis zeigen jedoch häufig keine nachhaltigen Pflegeeffekte. Gel-Formulierungen sind außerdem salzempfindlich, so dass sie beim Auftragen auf die Haut durch die Präsenz von Salzen häufig brechen.

Des Weiteren fühlen sich Hydrodispersionen im Vergleich zu Emulsionen häufig klebrig an. Ein weiterer Nachteil von Hydrodispersionen, insbesondere solcher, die Acrylate als Gelbildner enthalten, ist der unvorteilhafte Abrieb, wenn die auf die Haut aufgetragene Hydrogelschicht leicht und in unschöner Weise ("Röllchenbildung") abgetragen wird.

Nachteil der Hydrodispersionen des Standes der Technik ist ferner, dass diese bei einem höheren Gehalt an Lipiden (größer 4 Gew.-%) zur Phasentrennung neigen, die insbesondere bei höheren Temperaturen (≥ 40 °C) auftreten kann.

In der WO 2005097057 A1 werden Gelformulierungen offenbart, die polymere Gelbbildnern, Wasser, Öle sowie mindestens ein Wachs, das einen Schmelzpunkt von 30°C und mehr besitzt, umfassen. Die Formulierungen sind frei von kationischen oder anionischen Emulgatoren oder Tensiden.

In der WO 2005097055 A1 werden Gelformulierungen beschrieben, die polymere Gelbildner, Wasser, eine bei 25°C flüssige Ölphase und mindestens eine Wachskomponente mit einem Schmelzpunkt von wenigstens 30°C ausgewählt aus der Gruppe der Pentaerythritester, der Dipentaerythritester und/oder der Tripentaerythritester, umfassen.

Die sensorischen Eigenschaften dieser Gelformulierungen stellen sich jedoch als nicht so attraktiv dar. Der Rückstand fühlte sich sehr wachsig an.

Wünschenswert ist es daher Hydrodispersionen bereit zu stellen, die sensorisch verbesserte Eigenschaften aufweisen und insbesondere deren Rückstand weniger wachsig erscheint.

Unter Kosmetik kann man alle Maßnahmen zusammenfassen, die aus ästhetischen Gründen Veränderungen an Haut und Haaren vornehmen oder zur Körperreinigung angewendet werden. Kosmetik bedeutet also, das Körperäußere zu pflegen, zu verbessern und zu verschönern, um auf sichtbare, fühlbare und riechbare Weise sowohl den Mitmenschen als auch sich selbst zu gefallen. Schon vor Jahrtausenden wurde Kosmetik vom Menschen zu diesem Zweck angewandt. Man färbte Lippen und Gesicht, salbte sich mit wertvollen Ölen und badete in duftendem Wasser.

Eine für den Verbraucher sehr wesentliche, dabei aber nur schwer quantitativ messbare Eigenschaft kosmetischer Produkte ist ihre Textur. Unter dem Begriff "Textur" werden diejenigen Eigenschaften eines Kosmetikums verstanden, die auf den Gefügebau der Zubereitung zurückgehen, durch Tast- und Berührungssinne empfunden und ggf. in mechanischen oder rheologischen Fließeigenschaften ausgedrückt werden können. Die Textur kann insbesondere mittels Sensorik getestet werden. Die gegebenenfalls mit Hilfe von Zusatzstoffen beeinflussbare Textur kosmetischer Produkte ist für den Verbraucher von nahezu gleicher Bedeutung wie deren objektiv feststellbaren Wirkungen.

Mit dem Begriff "Sensorik" wird die wissenschaftliche Disziplin bezeichnet, die sich mit der Bewertung von kosmetischen Zubereitungen auf Grund von Sinneseindrücken befasst. Die sensorische Beurteilung eines Kosmetikums erfolgt anhand der visuellen, olfaktorischen und haptischen Eindrücke.
- *Visuelle Eindrücke:* alle mit dem Auge wahrnehmbaren Merkmale (Farbe, Form, Struktur).
- *Olfaktorische Eindrücke:* alle beim Einziehen von Luft durch die Nase wahrnehmbaren Geruchseindrücke, die häufig in Anfangsgeruch (Kopfnote), Hauptgeruch (Mittelnote, Körper) und Nachgeruch (Ausklang) differenziert werden können. Auch die erst bei der Anwendung freigesetzten flüchtigen Stoffe tragen zum olfaktorischen Eindruck bei.
- *Haptische Eindrücke:* alle Empfindungen des Tastsinns, die vornehmlich Gefüge und Konsistenz des Produktes betreffen.

Die sensorische Analyse macht von der Möglichkeit Gebrauch, den sensorischen Gesamteindruck eines Produktes integral zu erfassen. Nachteile der sensorische Analyse sind die Subjektivität des Eindrucks, eine leichte Beeinflussbarkeit der Prüfpersonen und die dadurch bedingte starke Streuung der Ergebnisse. Diesen Schwächen begegnet man heute durch den Einsatz von Gruppen geschulter Prüfpersonen, gegenseitige Abschirmung der Prüfer sowie statistische Auswertung der meist zahlreichen Analysendaten.

Aufgabe war es kosmetische oder dermatologische Hydrodispersionen bereit zu stellen, die mit Wachsen formuliert, ein angenehmes, sensorisch leichtes Profil und gute Pflegeeffekte aufweisen und bei Applikation auf der Haut nicht brechen.

Überraschenderweise wurde nun herausgefunden, dass die Sensorik der Formulierung positiv beeinflusst wird, wenn die Wachskombination so ausgewählt wird, dass sie mindestens eine Wachskomponente enthält, die einen Schmelzpunkt Tₒₙₛₑₜ kleiner 30°C hat und die gesamte Formulierung vorteilhaft mindestens einen Schmelzbereich mittels DSC zwischen 5°C und 30°C aufweist.

Die Erfindung ist eine Hydrodispersion umfassend
a.) Wasser, vorteilhaft 60 - 95 Gew.%, insbesondere 70 - 85 Gew.%,
b.) 0,05 - 5 Gew.% wenigstens eines Verdickungsmittels,
c.) 2 bis 20 Gew.% eines oder mehrerer Wachse,
d.) mehr als 0,5 Gew.% bis 20 Gew.% eines oder mehrerer Öle, vorteilhaft 1 bis 10 Gew.%, jeweils bezogen auf die Gesamtmasse der Dispersion,
wobei mindestens ein Wachs einen Schmelzpunkt Tₒₙₛₑₜ < 30°C aufweist und ein Verdickungsmittel enthalten ist aus der Gruppe der Homopolymere oder Copolymere von Acrylsäure und der Anteil an Emulgatoren bis zu 2 Gew.% beträgt.

Die Dispersion enthält bevorzugt keine anionischen und keine kationischen Emulgatoren und Tenside.

Die Dispersion umfasst vorteilhaft mindestens einen Schmelzbereich zwischen 5°C und 30°C nach DSC. Ein weiterer oder mehrere Schmelzbereiche können ausserhalb dieser Temperturspanne liegen.

Entgegen den Gelformulierungen des Standes der Technik sind die erfindungsgemäßen Hydrodispersionen sensorisch attraktiv und pflegend.

Die erfindungsgemäße Dispersion weist eine überraschend reichhaltige Textur bei der Entnahme auf.

Aufgrund der erfindungsgemäßen Wachsschmelzkaskade zeigen die Zubereitungen zudem einen Kühleffekt beim Verteilen und eine sehr leichte Verteilbarkeit auf der Haut.

Weitere Verdickungsmittel werden erfindungsgemäß vorteilhaft aus einer oder mehreren der folgenden Gruppen gewählt:
- Acrylamid,
- organische, natürliche Verbindungen, wie beispielsweise Agar-Agar, Carrageen, Tragant, Gummi arabicum, Alginate, Pektine, Polyosen, Guar-Mehl, Johannisbrotbaumkernmehl, Stärke, Dextrine, Gelatine, Casein, Xanthan Gum
- organische, abgewandelte Naturstoffe, wie z. B. Carboxymethylcellulose und andere Celluloseether, Hydroxyethyl- und -propylcellulose und mikrokristalline Cellulose oder dergleichen, .
- organische, vollsynthetische Verbindungen, wie z. B. Polyacryl- und Polymethacryl-Verbindungen, Vinylpolymere, Polycarbonsäuren, Polyether, Polyimine, Polyamide, Polyurethane, AMPS Copolymere wie z.B. Ammonium Acryloyldimethyltaurate/VP Copolymer und Sodium Acrylate/ Acryloyldimethyltaurate/Dimethylacrylamide Crosspolymer, Acrylic Acid/VP Crosspolymer, PVP, Acrylamide/AmmoniumAcrylate Copolymer.

Besonders bevorzugte Verdickungsmittel im Sinne der vorliegenden Erfindung sind Polyacrylate, d. h. Polymere auf Basis von Estern der Acrylsäure (Polyacrylsäureester) der allgemeinen Strukturformel 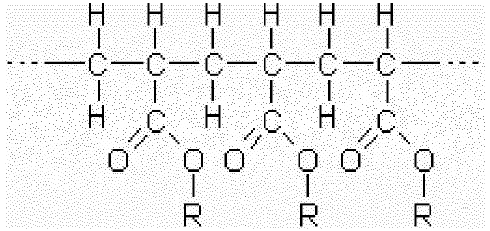 worin R für lineare, verzweigte oder cyclische, ggf. funktionelle Substituenten (z. B. Hydroxy-, Amin- oder Epoxid-Gruppen) enthaltende Alkyl-Reste steht, z. B. für Methyl-, Ethyl-, Isopropyl-, *tert*-Butyl-, Cyclohexyl-, 2-Ethylhexyl-, Dodecyl-, 2-Hydroxyethyl- und 2-Dimethylaminoethyl-.

Ganz besonders bevorzugt sind Polyacrylate mit der INCI-Bezeichnung Sodium Polyacrylate, beispielsweise das unter der Handelsbezeichnung Cosmedia SP bei der Fa. Cognis erhältliche.

Der Anteil an Verdickungsmitteln wird bevorzugt im Bereich von 0,1 bis 2 Gew.% gewählt, bezogen auf die Gesamtmasse der Zubereitung.

Werden einzelne oder mehrere Wachse eingesetzt, so beziehen sich die Anteilsangaben sowohl auf einzelne als auch auf mehrere der Wachse, sofern die Gesamtmasse die vorteilhafte Summe nicht überschritten wird.

Unter dem Begriff Wachs werden üblicherweise alle natürlichen oder künstlich gewonnenen Stoffe und Stoffgemische mit folgenden Eigenschaften verstanden:
Die erfindungsgemäßen Wachse sind bei Raumtemperatur (20°C) von fester bis brüchig harter Konsistenz, grob bis feinkristallin, durchscheinend bis trüb und schmelzen oberhalb von 20°C ohne Zersetzung. Sie sind schon wenig oberhalb des Schmelzpunktes niedrigviskos und nicht fadenziehend und zeigen eine starke temperaturabhängige Konsistenz und Löslichkeit. Erfindungsgemäß einsetzbar ist eine Wachskomponente oder ein Gemisch von Wachskomponenten, die bei einer Temperatur Tₒₙₛₑₜ<30°C anfängt zu schmelzen aber bei RT (20°C) noch fest ist/sind. D.h. mindestens ein Wachs weist einen Schmelzpunkt Tₒₙₛₑₜ < 30°C auf oder vorteilhaft alle gewählten Wachse weisen diesen Schmelzpunkt auf.
Tₒₙₛₑₜ wird mittels DSC bestimmt.

DSC (Differential Scanning Calorimetry) ist ein thermisches Verfahren zur Messung von abgegebener/aufgenommener Wärmemenge einer Probe bei isothermer Arbeitsweise, Aufheizung oder Abkühlung (siehe DIN 53765, DIN 51007, ASTM E 474, ASTM D 3418). DSC ist eine vergleichende Messmethode, die die Bestimmung von Wärmemengen physikalischer und chemischer Prozesse ermöglicht. Wenn ein Material seinen physikalischen Zustand ändert, wie z.B. Schmelzen oder Umwandlung einer Kristallform in eine andere oder wenn es chemisch reagiert, wird Wärme dabei aufgenommen oder abgegeben. Diese Wärmemengen sind mit Hilfe der DSC quantitativ messbar. Die Methode verläuft zyklisch, so dass nach der ersten Aufheizkurve ein definiertes Abkühlen stattfindet und anschließend die Probe noch einmal im angegebenen Temperaturbereich aufgeheizt wird. Man erhält somit zweierlei Informationen: In der ersten Aufheizkurve sind alle thermische Effekte inklusiv Vorgeschichte erkennbar. In der zweiten Aufheizkurve ist die Vorgeschichte eliminiert worden und das reine thermische Verhalten der Probe unter definierten Abkühlbedingungen ist auswertbar. Die Schmelztemperatur Tₒₙₛₑₜ der Wachse wird bei der zweiten Aufheizkurve ermittelt. Dagegen ist der Schmelzbereich der Hydrodispersion zwischen 5°C und 30°C nach DSC der in der ersten Aufheizkurve ermittelte Bereich.

Als Wachse können erfindungsgemäß auch Fette und fettähnlichen Substanzen mit wachsartiger Konsistenz eingesetzt werden, solange sie einem Tₒₙₛₑₜ < von 30°C haben. Hierzu gehören u.a. Fette (Triglyceride), Mono und Diglyceride, natürliche und synthetische Wachse, Fett-und Wachsalkohole, Ester von Fettalkoholen und Fettsäuren sowie Fettsäureamide oder beliebige Gemische dieser Substanz.

Besonders bevorzugt werden die Wachse aus der Gruppe der Fette, insbesondere aus:
- Shorea Stenoptera Seed Butter (Tₐₙₛₑₜ=14,9°C)
- Hydrogenated Vegetable Oil (Tₒₙₛₑₜ=18,9°C)
- Hydrogenated Coco-Glycerides (Tₒₙₛₑₜ=26,8°C)
- Butyrospermum Parkii Butter (Tₐₙₛₑₜ=28,3°C)
- Theobroma Cacao (Cocoa) Seed Butter (Tₐₙₛₑₜ=14,5°C)
- Mango Butter (Tₒₙₛₑt=19,4°C)
- Hydrogenated Palm Kernel Glycerides and Hydrogenated Palm Glycerides (Lipocire A, Gattefosse) (Tₒₙₛₑₜ=20,3°C)
- Acacia Decurrens/Jojoba/Sunflower Seed Wax Polyglyceryl-3 Esters (Tₐₙₛₑₜ=14,4°C) aus der Gruppe der Ester aus Fettsäuren, insbesondere aus
- Cetearyl Nonanoate (Tₒₙₛₑₜ=24°C)
- Methyl Palmitate (Tₒₙₛₑₜ=27,7°C),
   sowie aus der Grupper der Fettalkohole, insbesondere aus
- Cetearyl Alcohol (Tₒₙₛₑₜ=28,1°C)
   gewählt.

Weiterer erfindungswesentlicher Aspekt ist, dass die Gesamtzubereitung mindestens einen Schmelzbereich zwischen 5°C und 30°C nach DSC aufweist.

Nachfolgend sind DSC-Vergleichsmessungen dargestellt, die den erfindunsgemäßen Einfluss des Schmelzbereiches der Hydrodsipersionen veranschaulichen.

Es wurden dazu folgende Vergleichszubereitungen getestet:

| A (Bespiel 2 aus EP1742607 B1) | |
|---|---|
| INCI | Gew.-% |
| Coco-Caprylate/Caprate | 5 |
| Dicaprylyl Carbonate | 5 |
| Pentaerythrityl Distearate | 1 |
| Sodium Polyacrylate | 1 |
| Aqua | 88 |

| B (Bespiel 6 aus WO 2005097057 A1) | |
|---|---|
| INCI | Gew.-% |
| Cyclomethicone | 10 |
| Pentaerythrityl Distearate | 1 |
| Sodium Polyacrylate | 1 |
| Aqua | 88 |

| C (Bespiel 4 aus EP1742607 B1) | |
|---|---|
| INCI | Gew.-% |
| Coco-Caprylate/Caprate | 5 |
| Dicaprylyl Carbonate | 5 |
| Pentaerythrityl Distearate | 1 |
| Sodium Polyacrylate | 1 |
| Aqua | 73 |
| Alcohol Denat. | 15 |

| D (Beispiel 1) | |
|---|---|
| INCI | Gew.-% |
| Olus Oil | 3,2 |
| Candelilla Cera | 0,2 |
| Cetyl Palmitate | 1 |
| Butyrospermum Parkii Butter | 4 |
| Hydrogenated Vegetable Oil | 0,6 |
| Hydrogenated Coco-Glycerides | 5 |
| Zea Mays Oil + CI 40800 + Tocopherol | 0,0004 |
| Cyclomethicone + Dimethiconol | 0,9 |
| Parfum | 0,2 |
| Glycerin | 8,7 |
| Methylparaben | 0,15 |
| Phenoxyethanol | 0,3 |
| Sodium Polyacrylate | 1,5 |
| Aqua | 74,2496 |

| E (Beispiel 2) | |
|---|---|
| INCI | Gew.-% |
| Olus Oil | 3,2 |
| Candelilla Cera | 0,2 |
| Cetyl Palmitate | 1 |
| Methyl Palmitate | 5 |
| Butyrospermum Parkii Butter | 4 |
| Hydrogenated Vegetable Oil | 0,6 |
| Zea Mays Oil + CI 40800 + Tocopherol | 0,0004 |
| Cyclomethicone + Dimethiconol | 0,9 |
| Parfum | 0,2 |
| Glycerin | 8,7 |
| Methylparaben | 0,15 |
| Phenoxyethanol | 0,3 |
| Sodium Polyacrylate | 1,5 |
| Aqua | 74,2496 |

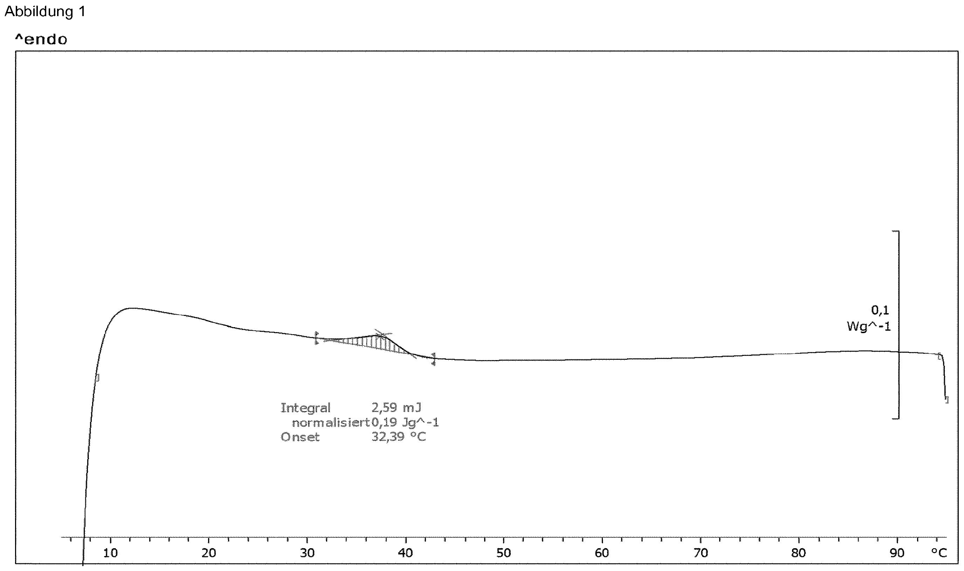

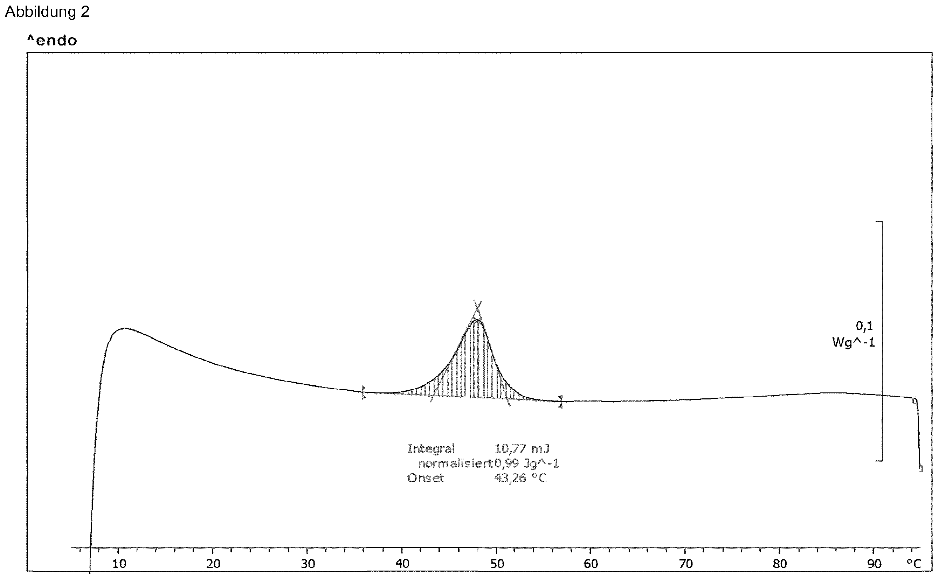

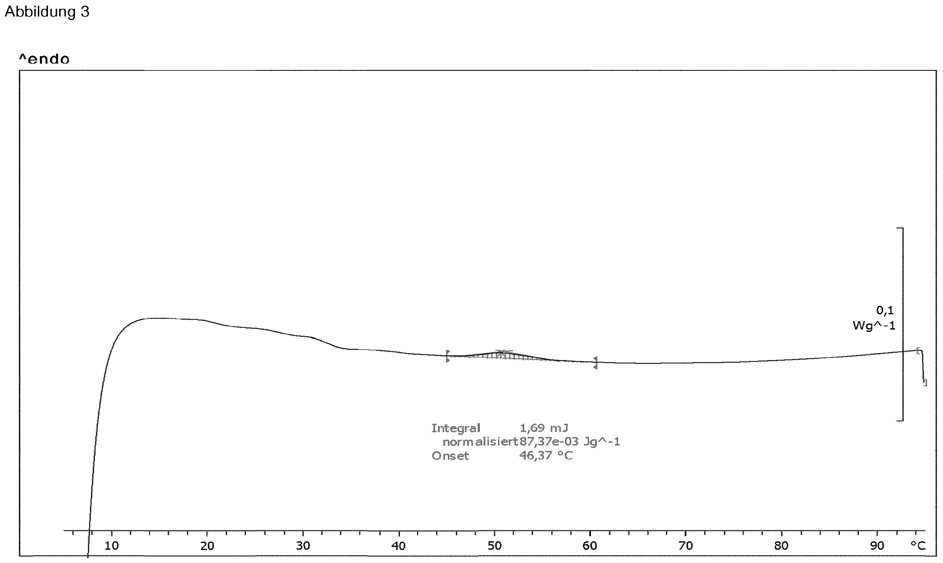

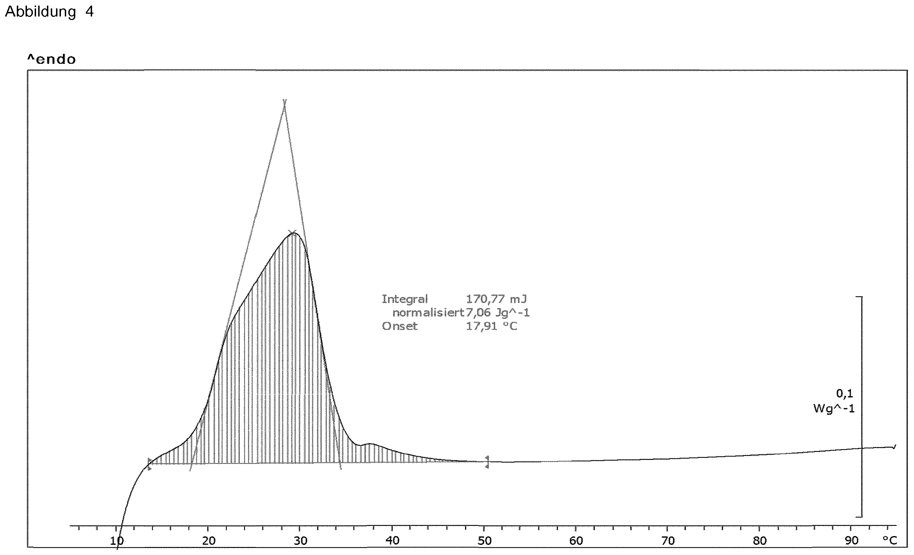

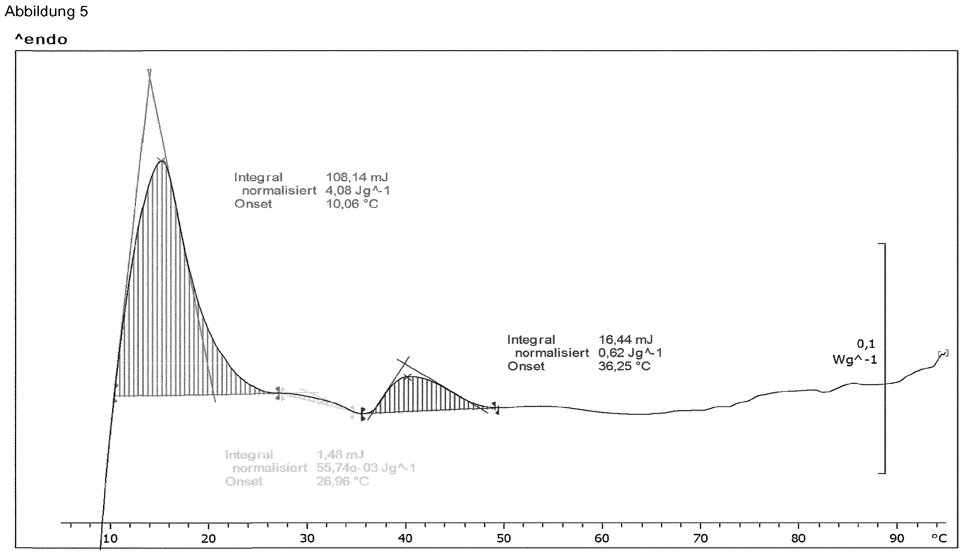

In den Abbildungen 1 bis 5 sind die DSC-Messergebnisse der Zubereitungen A bis E wiedergegeben.

Die "endo"-Darstellung der in den Abbildungen 1 bis 5 dargestellten DSC-Messungen ermöglicht die endothermen Prozeße positiv (nach oben) abzubilden. Die Kurven nach oben stellen dar, dass etwas schmilzt. Die jeweiligen Schmelzpeaks sind so ausgewertet, dass die Schmelzenthalpie dargestellt ist (Integral unnormiert und normiert) sowie Tₒₙₛₑₜ als Schmelztemperaturanfang des Peaks.

Die Zubereitungen des Standes der Technik A, B und C weisen keine Schmelzpeaks zwischen 0°C und 30°C auf, d.h. die Dispersionen weisen keinen Schmelzbereich zwischen 5°C und 30°C nach DSC auf.

In nachfolgenden Sensoriktests zeigten diese Zubereitungen A, B, C sich nicht so angenehm wie die erfindungsgemäßen Rezepturen D und E.

Die erfindungsgemäße Rezeptur D hat einen Peak bei 17,91 °C. Auf die Haut appliziert fühlt sie sich sehr angenehm an.

Die erfindungsgemäße Rezeptur E hat mehrere Peaks, unter anderen 2 Peaks zwischen 0 und 30°C. Auf die Haut applizier fühlt sie sich sehr angenehm an.

Die Beurteilung der Sensorik erfolgte im Labor bei n=4 geschulten Probanden.

Die erfindungsgemäßen Hydrodispersionen stellen bevorzugt kosmerische und/oder dermatologische Zubereitungen dar.

Stabile kosmetische Zubereitungen sind solche, die über einen längeren Zeitraum sowie bei Temperaturwechsel keinerlei Phasentrennung, Viskositätsänderungen, Koaleszenz, Ostwald-Reifung und/oder Aufrahmungen zeigen.

Eine Phasentrennung der Zubereitung zwischen Lipdphase und Wasserphase, auch als Brechen bezeichnet, ist daher während der Lagerung und Anwendung nicht erwünscht.

Die erfindungsgemäßen Zubereitungen sind stabil und zeigen keinerlei Phasentrennung bei der Applikation auf die Haut.

Die erfindungsgemäßen Zubereitungen sind bevorzugt frei von anionischen und/oder kationischen Emulgatoren/Tenside.

Sind jedoch aufgrund von Rohstoffverunreinigungen oder sonstigen Einschleppungen anionische oder kationische emulgierende Stoffe oder waschaktive Substanzen in den Zubereitungen enthalten, so gelten diese Zubereitungen erfindungsgemäß immer noch als "frei von" Emulgatoren und Tensiden, sofern deren Anteil nicht über 0,1 Gew.%, bezogen auf die Gesamtmasse der Zubereitung, beträgt.

Verdickungsmittel, die u.U. auch emulgierende Eigenschaften haben können, werden jedoch nicht zu den auszuschließenden Emulgatoren gezählt, da deren Hauptfunktion nicht die eines Emulgators ist.

Werden Einschränkungen auf bevorzugte Anteilsbereiche einer oder mehrerer Inhaltsstoffe vorgenommen und/oder Einschränkungen auf bestimmte Inhaltsstoffe, so beziehen sich die bevorzugten Anteilsbereiche dann auf die ausgewählten Inhaltsstoffe.

Die erfindungsgemäßen Hydrodispersion kann neben den Wachsen auch andere Öle oder Lipide enthalten, vorteilhaft zu einem Anteil bis zu 10 Gew.%, bezogen auf die Gesamtmasse der Zubereitung. Dabei ist jedoch darauf zu achten, dass die Gesamtzubereitung mindestens einen Schmelzbereich im erfindungsgemäßen Bereich von 5°C bis 30°C aufweisen sollte.

Dies kann man das erreichen in dem man erfindungsgemäße Wachse wählt, die einen Tₒₙₛₑₜ<30°C haben, und indem man die erhaltene Dispersionen entsprechend testet.

Die Gesamtmenge der Wachse ist größer als die Gesamtmenge der Öle.

Zusätzliche Lipide bzw. Öle werden bevorzugt gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Sojaöl, Erdnußöl, Rapsöl, Arganöl, Mandelöl, Palmöl, Kokosöl, Rizinusöl, Weizenkeimöl, Traubenkernöl, Distelöl, Nachtkerzenöl, Macadamianußöl und dergleichen mehr.

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Octylpalmitat, Octylcocoat, Octylisostearat, Octyldodeceylmyristat, Octyldodecanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether (Cetiol OE) und/oder Dicaprylylcarbonat, beispielsweise das unter der Handelsbezeichnung Cetiol CC bei der Fa. Cognis erhältliche.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe, insbesondere Mineralöl, Vaseline (Petrolatum), Paraffinöl, Squalan und Squalen, Polyolefine, hydrogenierte Polyisobutene und Isohexadecan. Unter den Polyolefinen sind Polydecene die bevorzugten Substanzen.

Bevorzugt wird natürliches Öl, vegetable oil oder mineral oil, insbesondere Paraffinum Liquidum gewählt, insbesondere zu einem Anteil von jeweils 2 bis 4 Gew.%, bezogen auf die Gesamtmasse der Zubereitung.

Die erfindungsgemäßen Zubereitungen können optional einen Gehalt an cyclischen und/oder linearen Silikonölen aufweisen.

Vorteilhaft werden Cyclomethicone, insbesondere Octamethylcyclotetrasiloxan, Cyclomethicone D5 und/oder Cyclomethicone D6 als erfindungsgemäßes Silikonöl eingesetzt. Ein weiteres erfindungsgemäß vorteilhaftes Silikonöl ist Dimethicon (auch: Dimethylpolysiloxan bzw. Polydimethylsiloxan mit der INCI-Bezeichnung Dimethicone).

Aber auch andere Silikonöle sind vorteilhaft im Sinne der vorliegenden Erfindung zu verwenden, beispielsweise Hexamethylcyclotrisiloxan, Poly(methylphenylsiloxan), Phenyltrimethicon, Phenyldimethicon, Hexamethylcyclotrisiloxan, Polydimethylsiloxan, Poly(methylphenylsiloxan), Cetyldimethicon, Behenoxydimethicon.

Es ist aber auch vorteilhaft, Silikonöle ähnlicher Konstitution wie der vorstehend bezeichneten Verbindungen zu wählen, deren organische Seitenketten derivatisiert, beispielsweise polyethoxyliert und/oder polypropoxyliert sind. Dazu zählen beispielsweise Poly-siloxanpolyalkyl-polyethercopolymere wie das Cetyl-Dimethicon-Copolyol oder auch das Lauryl-Methicon-Copolyol.

Besonders vorteilhaft zur Verwirklichung der erfindungsgemäßen Textur wird eine Mischung von cyclischen und linearen Silikonölen, insbesondere von Cyclomethicon und Dimethicon, verwendet.

Der Anteil an ein oder mehreren Silikonöle, besonders bevorzugt Cyclomethicon und Dimethicon, wird bevorzug gewählt im Bereich von 0,1 bis 10 Gew.%, besonders 0,5 bis 8 Gew.%, jeweils bezogen auf das Gesamtgewicht der Zubereitung.

In einer besonders bevorzugten Ausführungsform enthalten die Zubereitungen im Sinne der vorliegenden Erfindung sogenannte Moisturizer. Als Moisturizer werden Stoffe oder Stoffgemische bezeichnet, welche kosmetischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhafte Moisturizer im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff, Glyceryl Glucoside.

Vorteilhaft umfasst die erfindungsgemäße Zubereitung Glyceryl Glucoside, insbesondere zu einem Ateil von 0,001% bis 8 Gew.%, bezogen auf die Gesamtmasse der Zubereitung.

Vorteilhaft wird die Menge an Moisturizern, eine oder mehrere Verbindungen, aus dem Bereich von 1 bis 20 Gew.-%, bevorzugt von 3 bis 15 Gew.-%, besonders bevorzugt von 5 bis 12 Gew.-% - jeweils bezogen auf das Gesamtgewicht der Zubereitung - gewählt.

Die kosmetische Zusammensetzungen im Sinne der vorliegenden Erfindung können, je nach ihrem Aufbau, beispielsweise verwendet werden als Hautschutzcreme, Reinigungsmilch, Sonnenschutzlotion, Nährcreme, Tages- oder Nachtcreme usw. Besonders bevorzugt im Sinne der vorliegenden Erfindung sind Zubereitungen zur, insbesondere großflächigen Anwendung im Körperpflegebereich.

Vorteilhaft wird die Hydrodsipersion oder Zubereitung als Hautpflegecreme verwendet.

Die Vorteile der erfindungsgemäßen Hydrodispersion sind deren leichte Verteilbarkeit, erfrischender Effekt bei der Applikation und dennoch eine pflegende Wirkung, die insbesondere durch die Wachse hervorgerufen werden.

Die erfindungsgemäße Hydrodisersion ist besonders hautverträglich, da wenig bzw. keine Öle, insbesondere keine spreitende Öle, wenig Emulgatoren und wenig Konservierungsmittel enthalten sind. Eine Anwendung der Hydrodispersion am Auge ist damit problemlos möglich.

Die Zusammensetzungen können auch UV-Filter enthalten gewählt aus der Gruppe der Verbindungen 2-Phenylbenzimidazol-5-sulfonsäure und/oder deren Salze, Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)ben zolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 4-(tert.-Butyl)-4'-methoxydibenzoylmethan; 2-Ethylhexyl-2-cyano-3,3-diphenylacrylat; 4-(Dimethylamino)-benzoesäure(2-ethylhexyl)ester; Diethylamino Hydroxybenzoyl Hexyl Benzoate, 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester;Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer;Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr.288254-16-0); 4,4',4"-(1,3,5-Triazin-2,4,6-triyltriimino)-tris-benzoesäure-tris(2-ethylhexylester)(auch: 2,4,6-Tris-[anilino-(p-carbo-2'-ethyl-1'-hexyloxy)]-1,3,5-triazin (INCI: Ethylhexyl Triazone);2,4-Bis-{[4-(2-ethyl-hexyloxy)-2-hydroxy]-phenyl}-6-(4-methoxyphenyl)-1,3,5-triazin; 2,4,6-Tribiphenyl-4-yl-1,3,5-triazin; Merocyanine, Butyl Methoxydibenzoylmethane, Octocrylene, sowie Titandioxide und Zinkoxide.

Die kosmetischen oder dermatologischen Zubereitungen gemäß der Erfindung können ferner kosmetische Hilfsstoffe und Wirkstoffe enthalten, wie sie üblicherweise in solchen Zubereitungen verwendet werden, z. B. Konservierungsmittel, Konservierungshelfer, Bakterizide, Substanzen zum Verhindern des Schäumens, Farbstoffe und Farbpigmente, Verdickungsmittel, anfeuchtende und/oder feuchthaltende Substanzen, Anti-Aging-Substanzen oder andere übliche Bestandteile einer kosmetischen oder dermatologischen Formulierung wie Polyole, Polymere, Schaumstabilisatoren, organische Lösungsmittel oder Silikonderivate, sofern der Zusatz die geforderten Eigenschaften hinsichtlich der Sensorik und Pflegeeffekte nicht beeinträchtigt. Folgende Wirkstoffe können z.B. enthalten sein, gewählt aus der Gruppe der Verbindungen Gylcyrrhetinsäure, Harnstoff, Arctiin, alpha-Liponsäure, Folsäure, Phytoen, D-Biotin, Coenzym Q10, alpha-Glucosylrutin, Tocopheryl Acetate, Carnitin,Carnosin, Coffein, natürliche und/oder synthetische Isoflavonoide, Glycerylglucose, Kreatin,Kreatinin, Taurin, Magnolia, β-Alanin und/oder Licochalcon A.

Nachfolgende Beispiele erläutern die erfindungsgemäßen Hydrodispersionen. Die angeführten Zahlen sind, sofern nichts anderes angegeben, Gewichtsanteile bezogen auf die Gesamtmasse der Zubereitung.

### Beispiele 1 und 2:

| INCI | Rezeptur 1 | Rezeptur 2 |
|---|---|---|
| Olus Oil | 3,2 | 3,2 |
| Candelilla Cera | 0,2 | 0,2 |
| Cetyl Palmitate | 1 | 1 |
| Methyl Palmitate | 5 | **0** |
| Hydrogenated Coco-Glycerides | **0** | 5 |
| Butyrospermum Parkii Butter | 4 | 4 |
| Hydrogenated Vegetable Oil | 0,6 | 0,6 |
| Cyclomethicone + Dimethiconol | 0,9 | 0,9 |
| Parfum | 0,2 | 0,2 |
| Glycerin | 8,7 | 8,7 |
| Zea Mays Oil + CI 40800 + Tocopherol | 0,0004 | 0,0004 |
| Methylparaben | 0,15 | 0,15 |
| Phenoxyethanol | 0,3 | 0,3 |
| Sodium Polyacrylate | 1,5 | 1,5 |
| Aqua | 74,2496 | 74,2496 |

Die erfindungsgemäßen Hydrodispersionen zeigen ein angenehmes, sensorisch leichtes Profil und gute Pflegeeffekte. Weiterhin brechen die Formulierungen bei Applikation auf der Haut nicht.

## Patentansprüche

1. Hydrodispersion umfassend
a) Wasser,
b) 0,05 - 5 Gew.% wenigstens eines Verdickungsmittels,
c) 2 bis 20 Gew.% eines oder mehrerer Wachse und
d) mehr als 0,5 Gew.% bis 20 Gew.% eines oder mehrerer Öle neben den Wachsen, jeweils bezogen auf die Gesamtmasse der Dispersion,
**dadurch gekennzeichnet, dass**
- mindestens ein Wachs einen Schmelzpunkt Tₒₙₛₑₜ kleiner 30°C aufweist,
- ein Verdickungsmittel gewählt wird aus der Gruppe der Homopolymere oder Copolymere von Acrylsäure,
- der Anteil an Emulgatoren bis zu 2 Gew.% beträgt und
- die Gesamtmenge der Wachse größer ist als die Gesamtmenge der Öle.

2. Hydrodispersion nach Anspruch 1 **dadurch gekennzeichnet, dass** der Anteil an Emulgatoren nicht über 0,1 Gew.% beträgt.

3. Hydrodispersion nach Anspruch 1 oder 2 **dadurch gekennzeichnet, dass** die Dispersion mindestens einen Schmelzbereich zwischen 5°C und 30°C nach DSC aufweist.

4. Hydrodispersion nach Anspruch 1, 2 oder 3 **dadurch gekennzeichnet, dass** weitere Verdickungsmittel ausgewählt werden aus der Gruppe
• Acrylamid,
• der organischen, natürlichen Verbindungen, wie insbesondere Agar-Agar, Carrageen, Tragant, Gummi arabicum, Alginate, Pektine, Polyosen, Guar-Mehl, Johannisbrotbaumkernmehl, Stärke, Dextrine, Gelatine, Casein, Xanthan Gum,
• der organischen, abgewandelten Naturstoffe, wie insbesondere Carboxymethylcellulose, Hydroxyethyl- und/oder -propylcellulose, mikrokristalline Cellulose, und/oder
• der organischen, vollsynthetischen Verbindungen, wie Vinylpolymere, Polycarbonsäuren, Polyether, Polyimine, Polyamide und/oder Polyurethane, AMPS Copolymere wie z.B. Ammonium Acryloyldimethyltaurate/VP Copolymer und PVP.

5. Hydrodispersion nach Anspruch 1 **dadurch gekennzeichnet, dass** als eines oder einziges der Verdickungsmittel Natriumpolyacrylat gewählt wird.

6. Hydrodispersion nach einem der vorstehenden Ansprüche **dadurch gekennzeichnet, dass** der Anteil an Verdickungsmitteln im Bereich 0,1 bis 2 Gew.%, bezogen auf die Gesamtmasse der Hydrodispersionszubereitung, gewählt wird.

7. Hydrodispersion nach einem der vorstehenden Ansprüche **dadurch gekennzeichnet, dass** der Anteil an Wasser gewählt wird im Bereich von 60 bis 95 Gew.%, insbesondere 70 bis 85 Gew.%, bezogen auf die Gesamtmasse der Hydrodispersionszubereitung.

8. Hydrodispersion nach einem der vorstehenden Ansprüche **dadurch gekennzeichnet, dass** der oder die Wachse mit einem Tₒₙₛₑₜ unterhalb von 30°C gewählt werden aus der Gruppe der Fette (Triglyceride), Mono- und Diglyceride, natürliche und synthetische Wachse, Fett- und Wachsalkohole, Fettsäuren, Ester von Fettalkoholen und Fettsäuren sowie Fettsäureamide oder beliebige Gemische dieser Substanzen.

9. Hydrodispersion nach Anspruch 8 **dadurch gekennzeichnet, dass** der oder die Wachse gewählt werden aus der Gruppe Shorea Stenoptera Seed Butter, Hydrogenated Vegetable Oil, Hydrogenated Coco-Glycerides, Butyrospermum Parkii Butter, Theobroma Cacao (Cocoa) Seed Butter, Mangobutter, Methyl Palmitat ,Hydrogenated Palm Kernel Glycerides and Hydrogenated Palm Glycerides (Lipocire A, Gattefossé) , Acacia Decurrens/Jojoba/Sunflower Seed Wax Polyglyceryl-3 Esters , Cetearyl Nonanoate und/oder Cetearyl Alcohol.

10. Hydrodispersion nach einem der vorstehenden Ansprüche umfassend 1 bis 10 Gew.%, jeweils bezogen auf die Gesamtmasse der Dispersion, an einem oder mehreren Ölen.

11. Hydrodispersion nach einem der vorstehenden Ansprüche **dadurch gekennzeichnet, dass** die Dispersion keine anionischen Emulgatoren und Tenside enthält.

12. Hydrodispersion nach einem der vorstehenden Ansprüche **dadurch gekennzeichnet, dass** die Dispersion keine kationischen Emulgatoren und Tenside enthält.

13. Kosmetische oder dermatologische Zubereitung umfassend oder bestehend aus einer Hydrodispersion nach einem der Ansprüche 1 bis 12.

14. Verwendung der Hydrodsipersion oder Zubereitung nach einem der Ansprüche 1 bis 13 als Hautpflegecreme.

15. Verwendung nach Anspruch 14 zur Anwendung am Auge.

## Claims

1. Hydrodispersion comprising
a) water,
b) 0.05%-5% by weight of at least one thickener,
c) 2% to 20% by weight of one or more waxes and
d) more than 0.5% to 20% by weight of one or more oils as well as the waxes, based in each case on the total mass of the dispersion,
**characterized in that**
- at least one wax has a melting point Tₒₙₛₑₜ of less than 30°C,
- a thickener is chosen from the group of the homopolymers or copolymers of acrylic acid,
- the proportion of emulsifiers is up to 2% by weight and
- the total amount of waxes is greater than the total amount of oils.

2. Hydrodispersion according to Claim 1, **characterized in that** the proportion of emulsifiers does not exceed 0.1% by weight.

3. Hydrodispersion according to Claim 1 or 2, **characterized in that** the dispersion has at least one melting range between 5°C and 30°C by DSC.

4. Hydrodispersion according to Claim 1, 2 or 3, **characterized in that** further thickeners are selected from the group of
• acrylamide,
• the organic natural compounds such as, in particular, agar-agar, carrageenan, tragacanth, gum arabic, alginates, pectins, polyoses, guar flour, carob seed flour, starch, dextrins, gelatins, casein, xanthan gum,
• the organic modified natural substances such as, in particular, carboxymethyl cellulose, hydroxyethyl and/or hydroxypropyl cellulose, microcrystalline cellulose, and/or
• the organic fully synthetic compounds such as vinyl polymers, polycarboxylic acids, polyethers, polyimines, polyamides and/or polyurethanes, AMPS copolymers, for example Ammonium Acryloyl-dimethyltaurate/VP Copolymer and PVP.

5. Hydrodispersion according to Claim 1, **characterized in that** sodium polyacrylate is chosen as one of the thickeners or the only thickener.

6. Hydrodispersion according to any of the preceding claims, **characterized in that** the proportion of thickeners is chosen in the range of 0.1% to 2% by weight, based on the total mass of the hydrodispersion formulation.

7. Hydrodispersion according to any of the preceding claims, **characterized in that** the proportion of water is chosen within the range from 60% to 95% by weight, especially 70% to 85% by weight, based on the total mass of a hydrodispersion formulation.

8. Hydrodispersion according to any of the preceding claims, **characterized in that** the wax(es) having a Tₒₙₛₑₜ below 30°C are chosen from the group of the fats (triglycerides), mono- and diglycerides, natural and synthetic waxes, fatty alcohols and wax alcohols, fatty acids, esters of fatty alcohols and fatty acids, and fatty acid amides, or any desired mixtures of these substances.

9. Hydrodispersion according to Claim 8, **characterized in that** the wax(es) are chosen from the group of Shorea stenoptera seed butter, hydrogenated vegetable oil, hydrogenated coco-glycerides, Butyrospermum parkii butter, Theobroma cacao (cocoa) seed butter, mango butter, methyl palmitate, hydrogenated palm kernel glycerides and hydrogenated palm glycerides (Lipocire A, Gattefossé), Acacia decurrens/jojoba/sunflower seed wax polyglyceryl-3 esters, cetearyl nonanoate and/or cetearyl alcohol.

10. Hydrodispersion according to any of the preceding claims, comprising 1% to 10% by weight, based in each case on the total mass of the dispersion, of one or more oils.

11. Hydrodispersion according to any of the preceding claims, **characterized in that** the dispersion does not contain any anionic emulsifiers and surfactants.

12. Hydrodispersion according to any of the preceding claims, **characterized in that** the dispersion does not contain any cationic emulsifiers and surfactants.

13. Cosmetic or dermatological formulation comprising or consisting of a hydrodispersion according to any of Claims 1 to 12.

14. Use of the hydrodispersion or formulation according to any of Claims 1 to 13 as skincare cream.

15. Use according to Claim 14 for application to the eye.

## Revendications

1. Hydrodispersion, comprenant :
a) de l'eau,
b) 0, 05 à 5 % en poids d'au moins un épaississant,
c) 2 à 20 % en poids d'une ou de plusieurs cires, et
d) plus de 0,5 à 20 % en poids d'une ou de plusieurs huiles en plus des cires, à chaque fois par rapport à la masse totale de la dispersion,
**caractérisée en ce que**
- au moins une cire présente un point de fusion Tₒₙₛₑₜ inférieur à 30 ° C,
- un épaississant est choisi dans le groupe des homopolymères ou copolymères de l'acide acrylique,
- la proportion d'émulsifiants est de jusqu'à 2 % en poids et
- la quantité totale des cires est plus grande que la quantité totale des huiles.

2. Hydrodispersion selon la revendication 1, **caractérisée en ce que** la proportion d'émulsifiants n'est pas supérieure à 0,1 % en poids.

3. Hydrodispersion selon la revendication 1 ou 2, **caractérisée en ce que** la dispersion présente au moins une plage de fusion comprise entre 5 °C et 30 °C selon une DSC.

4. Hydrodispersion selon la revendication 1, 2 ou 3, **caractérisée en ce que** d'autres épaississants sont choisis dans le groupe composé par :
- l'acrylamide,
- les composés organiques, naturels, tels que notamment l'agar-agar, le carraghénane, la gomme adragante, la gomme arabique, les alginates, les pectines, les polyoses, la farine de guar, la farine de caroube, les amidons, la dextrine, la gélatine, la caséine, la gomme xanthane,
- les substances naturelles organiques, modifiées, telles que notamment la carboxyméthylcellulose, l'hydroxyéthylcellulose et/ou l'hydroxypropylcellulose, la cellulose microcristalline, et/ou
- les composés organiques, entièrement synthétiques, tels que les polymères de vinyle, les poly(acide carboxylique), les polyéthers, les polyimines, les polyamides et/ou les polyuréthanes, les copolymères d'AMPS, tels que par exemple le copolymère d'acryloyldimèthyltaurate d'ammonium/VP et la PVP.

5. Hydrodispersion selon la revendication 1, **caractérisée en ce que** le poly(acrylate de sodium) est choisi en tant qu'un des épaississants ou en tant qu'épaississant unique.

6. Hydrodispersion selon l'une quelconque des revendications précédentes, **caractérisée en ce que** la proportion d'épaississants est choisie dans la plage de 0,1 à 2 % en poids, par rapport à la masse totale de la préparation d'hydrodispersion.

7. Hydrodispersion selon l'une quelconque des revendications précédentes, **caractérisée en ce que** la proportion d'eau est choisie dans la plage de 60 à 95 % en poids, notamment de 70 à 85 % en poids, par rapport à la masse totale de la préparation d'hydrodispersion.

8. Hydrodispersion selon l'une quelconque des revendications précédentes, **caractérisée en ce que** la ou les cires ayant une Tₒₙₛₑₜ inférieure à 30 °C sont choisies dans le groupe constitué par les graisses (triglycérides), les monoglycérides et diglycérides, les cires naturelles et synthétiques, les alcools gras et les alcools de cire, les acides gras, les esters d'alcools gras et d'acides gras, ainsi que les amides d'acides gras ou les mélanges quelconques de ces substances.

9. Hydrodispersion selon la revendication 8, **caractérisée en ce que** la ou les cires sont choisies dans le groupe constitué par Shorea Stenoptera Seed Butter, Hydrogenated Vegetable Oil, Hydrogenated Coco-Glycerides, Butyrospermum Parkii Butter, Theobroma Cacao (Cocoa) Seed Butter, Mangobutter, Methyl Palmitat, Hydrogenated Palm Kernel Glycerides and Hydrogenated Palm Glycerides (Lipocire A, Gattefossé), Acacia Decurrens/Jojoba/Sunflower Seed Wax Polyglyceryl-3 Ester, Cetearyl Nonanoate et/ou Cetearyl Alcohol.

10. Hydrodispersion selon l'une quelconque des revendications précédentes, comprenant 1 à 10 % en poids, à chaque fois par rapport à la masse totale de la dispersion, d'une ou de plusieurs huiles.

11. Hydrodispersion selon l'une quelconque des revendications précédentes, **caractérisée en ce que** la dispersion ne contient pas d'émulsifiants et tensioactifs anioniques.

12. Hydrodispersion selon l'une quelconque des revendications précédentes, **caractérisée en ce que** la dispersion ne contient pas d'émulsifiants et tensioactifs cationiques.

13. Préparation cosmétique ou dermatologique comprenant ou constituée par une hydrodispersion selon l'une quelconque des revendications 1 à 12.

14. Utilisation de l'hydrodispersion ou de la préparation selon l'une quelconque des revendications 1 à 13 en tant que crème pour le soin de la peau.

15. Utilisation selon la revendication 14 pour une utilisation à l'œil.
